# EUROPÄISCHE PATENTSCHRIFT

(11) **EP 3 261 691 B1**
(45) Veröffentlichungstag und Bekanntmachung des Hinweises auf die Patenterteilung: **06.01.2021**
(21) Anmeldenummer: 16703904.9
(22) Anmeldetag: 28.01.2016
(51) Int. Cl.: A61M 5/24

(54) **KARPULENHALTER ZUR AUFNAHME EINER KARPULE UND ZUR BEFESTIGUNG AN EINE ANTRIEBS- UND/ODER AN EINE DOSIEREINRICHTUNG UND/ODER AN EINEM GEHÄUSE EINER INJEKTIONSVORRICHTUNG**
CARPULE HOLDER FOR RECEIVING A CARPULE AND FOR FASTENING TO A DRIVE MECHANISM AND/OR TO A DOSING MECHANISM AND/OR TO A HOUSING OF AN INJECTION DEVICE
SUPPORT DE CARPULE DESTINÉ À RECEVOIR UNE CARPULE ET À ÊTRE FIXÉ À UN SYSTÈME D'ENTRAÎNEMENT ET/OU À UN DISPOSITIF DE DOSAGE ET/OU À UN BOÎTIER D'UN DISPOSITIF D'INJECTION

(30) Priorität: 26.02.2015 CH 2602015
(43) Veröffentlichungstag der Anmeldung: 03.01.2018
(73) Patentinhaber: Ypsomed AG, 3401 Burgdorf (CH)
(72) Erfinder: HIRSCHEL, Jürg, 3007 Bern (CH); MOSER, Ulrich, 3412 Heimiswil (CH); BURREN, Stefan, 3150 Schwarzenburg (CH); BOHNER, Reto, 4537 Wiedlisbach (CH); BIGLER, Bernhard, 3362 Niederönz (CH); SCHRUL, Christian, 3400 Burgdorf (CH)
(74) Vertreter: Kleiner, Stefan
(86) Internationale Anmeldenummer: PCT/CH2016/000021
(87) Internationale Veröffentlichungsnummer: WO 2016/134491

(56) Entgegenhaltungen:
- WO-A1-01/60311
- WO-A1-2010/043533
- WO-A1-2014/066256
- US-A- 1 718 600
- US-A- 1 718 602
- US-A1- 2007 021 718

## Beschreibung

Die vorliegende Erfindung bezieht sich auf einen Karpulenhalter zur Aufnahme einer Karpule und zur Befestigung an eine Antriebs- und/oder an eine Dosiereinrichtung und/oder an einem Gehäuse einer Injektionsvorrichtung, auf eine Injektionsvorrichtung zur Verabreichung eines fluiden Produkts, insbesondere eines Medikaments, wie beispielsweise Teriparatid zur Osteoporosetherapie, und auf ein Verfahren zur Montage einer Karpule in einen Karpulenhalter. Die mit dem Karpulenhalter verbundene Injektionsvorrichtung kann zur Aufdosierung und zur Ausschüttung einer festgelegten Dosis benutzt werden.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oligonukleotide, Antikörper oder Teile von Antikörpern sowie geeignete Basis-, Hilfs- und Trägerstoffe.

Aus der WO2012/017035A1 ist ein Karpulenhalter für eine Medikamenten-Verabreichungsvorrichtung bekannt, wobei die Karpule mit Hilfe eines Klemmelements in dem Karpulenhalter befestigt is Auch aus der US 2007/021718, WO 01/60311, WO 2010/043533 und WO 2014/066256 sind ähnliche Karpulenhalter bekannt.

Es ist eine Aufgabe der vorliegenden Erfindung einen alternativen Karpulenhalter zur Aufnahme einer Karpule bereitzustellen, wobei eine Karpule an den Karpulenhalter befestigt werden kann, insbesondere spielfrei gehalten werden kann, um zur Dosiergenauigkeit des Injektionsgeräts beizutragen.

Ferner ist es eine Aufgabe der vorliegenden Erfindung ein Verfahren zur Montage einer Karpule an oder in einen Karpulenhalter bereitzustellen, wobei insbesondere die Karpule spielfrei in dem Karpulenhalter gehalten wird.

Diese Aufgaben werden durch die Gegenstände der unabhängigen Ansprüche gelöst. Vorteilhafte Ausführungsformen ergeben sich aus den abhängigen Ansprüchen.

Im Folgenden bedeutet distal eine Richtung zum injektionsnadelseitigen Ende des Karpulenhalters hin und proximal zum antriebs- und/oder dosiereinrichtungsseitigen und/oder gehäuseseitigen Ende hin.

Die Erfindung betrifft einen Karpulenhalter zur Aufnahme einer Karpule und zur Befestigung an eine Antriebs- und/oder an eine Dosiereinrichtung und/oder an einem Gehäuse einer Injektionsvorrichtung. Ferner weist der Karpulenhalter ein Halteelement zur Befestigung der Karpule an oder in dem Karpulenhalter auf, wobei das Halteelement derart ausgebildet ist, dass es mit dem Karpulenhalter verbindbar ist und von dem Karpulenhalter vollständig aufnehmbar ist.

Das Halteelement ist ein Bestandteil des Karpulenhalters. Das Halteelement hat eine Halte- und/oder Fixier- und/oder Postionierungsfunktion. Das Halteelement kann vorzugsweise aus Kunststoff gebildet sein. Ferner kann das Halteelement elastisch verformbar ausgebildet sein. Das Halteelement kann eine Nocke umfassen. Diese Nocke kann radial nach aussen ragen. Ferner kann vorzugsweise das Halteelement ein Klemmelement aufweisen. Das Klemmelement kann sich radial nach innen erstrecken, um die in den Karpulenhalter eingesetzte Karpule, insbesondere ein proximales Ende der Karpule zu halten oder zu umgreifen oder einzuklemmen.

Der Karpulenhalter kann ein Karpulenhaltergehäuse, insbesondere ein zylinderförmiges Karpulenhaltergehäuse umfassen. Das Halteelement ist von dem Karpulenhaltergehäuse des Karpulenhalters vollständig aufnehmbar oder aufgenommen. Das Halteelement ist in dem Karpulenhaltergehäuse axial bewegbar gelagert. Alternativ kann das Karpulenhaltergehäuse eine andere Ausgestaltung aufweisen. Das Karpulenhaltergehäuse ist derart ausgebildet, dass in dem Karpulenhaltergehäuse eine Karpule aufgenommen werden kann.

Das Karpulenhaltergehäuse und das Halteelement des Karpulenhalters sind zweiteilig ausgebildet. Vorzugsweise sind das Karpulenhaltergehäuse und das Halteelement aus verschiedenen Materialien hergestellt. Das Karpulenhaltergehäuse und das Halteelement können aus Kunststoff hergestellt sein, wobei das Karpulenhaltergehäuse vorzugsweise unverformbar ist und das Halteelement vorzugweise elastisch verformbar ausgebildet ist. Alle Bestandteile des Halteelements können vorzugsweise elastisch verformbar ausgebildet sein.

Das Halteelement kann zur Halterung und/oder zur Fixierung und/oder zur Positionierung einer Komponente der Injektionsvorrichtung an oder in einer anderen Komponente dienen, indem das Halteelement in einem gespannten Zustand die eine der Komponenten in einer bestimmten Position relativ zu der anderen hält, beispielsweise die eine Komponente in einen Anschlagkontakt mit der anderen spannt. Das Halteelement kann somit eine Komponente der Injektionsvorrichtung in einer definierten Position in der Injektionsvorrichtung halten. Die definierte Position wird durch die örtliche Anordnung der zu haltenden Komponente relativ zu einer anderen Komponente der Injektionsvorrichtung bestimmt. Die Komponenten können eine Karpule in einem Karpulenhalter sein. Die Karpule kann vorzugsweise mittels Halteelement an oder in dem Karpulenhalter spielfrei gehalten werden, indem die Karpule in dem Karpulenhalter an der distalen Seite in axialen Anschlagkontakt und an der proximalen Seite mit Hilfe des Halteelements gehalten wird. Auf diese Weise können Fertigungs- und Montagetoleranzen ausgeglichen werden.

Das Halteelement kann ein Ringelement aufweisen. Das Ringelement kann ring- oder hülsenförmig ausgebildet sein. Das Ringelement kann ferner vorzugsweise zylinderförmig ausgebildet sein. An dem Ringelement können ein erster und ein zweiter Federsitz vorgesehen sein. Das Ringelement kann mehrere Bügel und mehrere Verbindungsstege umfassen, wobei der erste Federsitz über die mehreren Bügel und über die mehreren Verbindungsstege mit dem zweiten Federsitz verbunden sein können. Die Bügel und vorzugsweise die Verbindungsstege sind in axialer Richtung elastisch verformbar. Alternativ können die Verbindungsstege axial steif ausgebildet sein. Die Bügel erstrecken sich in Umfangsrichtung, wobei sie gegen distal oder gegen proximal gekrümmt ausgebildet sind. Der erste Federsitz kann über einen ersten Verbindungssteg mit einem ersten Bügel verbunden sein, wobei der erste Bügel über einen zweiten Verbindungssteg mit einem zweiten Bügel und der zweite Bügel über einen zweiten Verbindungssteg an dem zweiten Federsitz verbunden sein kann. Der erste und der zweite Bügel können elastische Biegearme bilden. Der erste und der zweite Bügel ragen somit über die Verbindungsstege in axialer Richtung von dem ersten und von dem zweiten Federsitz ab. Alternativ können auch eine andere Anzahl Bügel, Verbindungsstege und Federsitze an dem Halteelement vorgesehen sein. Ferner kann das Ringelement auch eine andere Ausgestaltung aufweisen, vorausgesetzt, dass das Ringelement längs der axialen Achse auf Druck beansprucht werden kann.

Der Karpulenhalter kann ferner in einer ersten Position, bei welcher die Karpule im Karpulenhalter bewegbar aufgenommen ist, und in einer zweiten Position, bei welcher die Karpule im Karpulenhalter aufgenommen und befestigt ist, das Halteelement zumindest teilweise in einem Ringspalt zwischen dem Karpulenhalter und der Karpule halten. Das Halteelement ist derart ausgebildet, dass das Halteelement in der zweiten Position die Karpule relativ zu dem Karpulenhalter axial spielfrei halten kann, Dabei kann die Karpule in distaler Richtung an oder in dem Karpulenhalter und in proximaler Richtung an dem Haltelement abgestützt sein.

In einem Ausführungsbeispiel kann ferner an dem Halteelement ein Vorsprung vorgesehen sein, welcher über ein Federelement mit dem Halteelement verbunden ist. Der Vorsprung kann radial nach aussen ragen, wobei das Klemmelement, welches ebenfalls an einem Federelement angeordnet ist, radial nach innen ragt. Ferner kann in der ersten Position der Vorsprung des Halteelements in die Ausnehmung des Karpulenhalters ragen, um das Halteelement mit dem Karpulenhalter zu verbinden. In der ersten Position ist das Halteelements in den Karpulenhalter eingesetzt, indem der Vorsprung des Halteelements in die Ausnehmung des Karpulenhalters eingerastet ist. Die Verbindung zwischen dem Vorsprung des Halteelements und der Ausnehmung des Karpulenhalters ist lösbar ausgebildet, In der zweiten Position wird der Vorsprung des Halteelements aus der Ausnehmung des Karpulenhalters gedrückt, wobei die an dem zweiten Federsitz angeordnete Nocke des Halteelement in die Ausnehmung des Karpulenhalters gelangt, um das Halteelement elastisch vorzuspannen. Die Verbindung zwischen der Nocke und der Ausnehmung des Karpulenhalters kann unlösbar ausgebildet sein. Die unlösbare Verbindung kann formschlüssig ausgebildet sein. Das Halteelement kann folglich in axialer und radialer Richtung elastisch vorgespannt werden. Die Nocke des Halteelements kann radial nach aussen ragen. Zudem kann das Klemmelement des Halteelements in der zweiten Position einen proximalen Karpulenrand der Karpule halten oder umgreifen. Die Karpule wird zwischen dem Karpulenhalter und dem Halteelement in dem Karpulenhalter spielfrei gehalten.

In einer Ausgestaltung ist das Halteelement in der ersten Position in dem Karpulenhaltergehäuse des Karpulenhalters eingesetzt, wobei das Halteelement in dem Karpulenhaltergehäuse derart angeordnet ist, dass eine Karpule in das Karpulenhaltergehäuse eingefügt werden kann. Der Karpulenhalter ist in der ersten Position somit, beispielsweise als Baugruppeneinheit, transportfähig, und es kann an einem anderen Ort, beispielsweise an einem Montageort, eine Karpule in den Karpulenhalter in der ersten Position eingesetzt werden. Der mit der Karpule eingesetzte Karpulenhalter kann beispielsweise an dem Montageort mittels einer in distaler Richtung wirkenden axialen Kraft, welche auf das Halteelement des Karpulenhalters wirkt, von der ersten Position in die zweite Position bewegt werden. In der zweiten Position ist die Karpule in dem Karpulenhalter fest, insbesondere axial spielfrei gehalten. Die Karpule kann nicht mehr aus dem Karpulenhalter genommen werden.

Das in dem Karpulenhaltergehäuse des Karpulenhalters aufgenommene Halteelement befindet sind in der zweiten Position relativ zu der ersten Position versetzt angeordnet. Eine axiale Kraft, welche auf einen Federsitz des Halteelements wirken kann, kann dazu führen, dass das Halteelement relativ zu dem Karpulenhaltergehäuse in axialer Richtung verschoben wird. Vorzugsweise wirkt die axiale Kraft in distaler Richtung derart, dass das Halteelement von der ersten Position in die zweite Position in distaler Richtung bewegt wird. Ein Werkzeug, beispielsweise eine Stoss- oder Schiebeeinrichtung kann auf einen Federsitz des Halteelements, insbesondere den zweiten Federsitz des Halteelements derart wirken, dass das Halteelement relativ zu dem Karpulenhaltergehäuse des Karpulenhalters in axialer Richtung bewegt wird.

In einem anderen Ausführungsbeispiel kann das Halteelement in ein mit einer Karpule aufgenommenen Karpulenhaltergehäuse des Karpulenhalters eingesetzt werden. Das Halteelement kann eine Nocke, welche an einem Federsitz, insbesondere dem zweiten Federsitz angeordnet ist, und ein Klemmelement, welches an einem Ringelement vorgesehen ist, umfassen. Um das Halteelement in den Karpulenhalter einzusetzen, so dass die Karpule in dem Karpulenhalter gehalten werden kann, kann die Nocke des Halteelements in eine Ausnehmung des Karpulenhaltergehäuses des Karpulenhalters einrasten, wobei ein Klemmelement einen proximalen Karpulenrand der Karpule hält oder umgreift. Die Nocke des Halteelements kann mit der Ausnehmung eine unlösbare Verbindung bilden, insbesondere formschlüssig ausgebildet sein. Das Halteelement wird dabei in axialer, insbesondere distaler Richtung vorgespannt, wobei das Halten der Karpule in dem Karpulenhalter durch eine Klemmpassung beziehungsweise durch einen Klemmsitz erfolgt.

In einem anderen Ausführungsbeispiel kann das Halteelement ebenfalls in ein mit einer Karpule aufgenommenen Karpulenhaltergehäuse des Karpulenhalters eingesetzt werden. Das Haltelement kann ein Ringelement in Form eines Zylinders umfassen. Das Ringelement weist folglich keinen Bügel, keinen Verbindungssteg und keinen Federsitz auf. Beim Einsetzen des Halteelements in einen eine Karpule aufgenommenen Karpulenhalter kann der Nocken des Halteelements in eine Ausnehmung des Karpulenhaltergehäuses des Karpulenhalters einrasten, wobei ein Klemmelement des Halteelements die Karpule an oder in den Karpulenhalter klemmt. Der Nocken des Halteelements kann in die Ausnehmung des Karpulenhalters rasten, wobei die Verbindung unlösbar, insbesondere formschlüssig ausgebildet ist.

In einer alternativen Ausführungsform kann die Karpule an oder in den Karpulenhalter geklebt oder geschweisst werden. In dieser Ausführungsform ersetzt der Klebstoff oder die Schweissung das Halteelement. Es ist insbesondere kein elastisch verformbar ausgebildetes Halteelement erforderlich. Wenn die Karpule in axialen, insbesondere in distalen Anschlagkontakt mit dem Karpulenhalter gelangt, wird die Karpule mit dem Karpulenhalter verklebt oder verschweisst. Die Verbindung zwischen Karpule und Karpulenhalter kann unlösbar ausgebildet sein. Die Klebe- oder Schweissverbindung zwischen der Karpule und dem Karpulenhalter kann kraftschlüssig ausgebildet sein.

In einer alternativen Ausführungsform kann die Karpule und in dem Karpulenhalter mit Hilfe eines Formschluss gehalten werden. Wenn beispielsweise die Karpule in axialen, insbesondere in distalen Anschlagkontakt mit dem Kapulenhalter ist, kann an einer Mantelinnenseite des Karpulenhaltergehäuses des Karpulenhalters ein nach innen ragendes Halteelement derart angebracht werden, dass die Karpule in dem Karpulenhalter gehalten, insbesondere spielfrei gehalten wird. Das Halteelement kann elastisch verformbar oder elastisch unverformbar ausgebildet sein. Das Halteelement kann unlösbar mit dem Karpulenhalter verbunden sein. Die Verbindung zwischen dem Halteelement und dem Karpulenhalter kann kraftschlüssig oder formschlüssig ausgebildet sein. Das Halteelement kann ein Klebstoff oder ein Befestigungsmittel sein. Vorzugsweise können das Halteelement ein oder mehrere Klebstofftropfen sein. Der oder die mehreren Klebstofftropfen können nach dem Einfügen der Karpule in den Karpulenhalter an der Mantelinnenseite des Karpulenhaltergehäuses des Karpulenhalters angebracht werden. Die oder die mehreren Klebstofftropfen sind vorzugsweise am proximalen Ende eines proximalen Karpulenrands der Karpule angeordnet. Der Klebstoff oder das Befestigungsmittel kann einen axialen, insbesondere einen proximalen Anschlagkontakt bildet. Alternativ kann der Klebstoff oder das Befestigungsmittel formschlüssig und kraftschlüssig die Karpule in dem Karpulenhalter halten, insbesondere spielfrei halten.

Ferner betrifft die Erfindung ein Verfahren zur Montage einer Karpule an oder in einen Karpulenhalter, wobei insbesondere die Karpule in dem Karpulenhalter spielfrei gehalten wird. Dabei kann zuerst ein Halteelement in den Karpulenhalter eingebracht werden. Der Karpulenhalter umfasst vorzugsweise ein Karpulenhaltergehäuse, in welchem eine Ausnehmung vorgesehen ist, wobei ein an dem Halteelement angebrachter Vorsprung in die Ausnehmung des Karpulenhalters einrastet. In dem mit dem Halteelement vorgesehenen Karpulenhalter kann die Karpule eingesetzt werden. Die Karpule gelangt an der distalen Seite in axialen Anschlagkontakt mit dem Karpulenhalter. Das Halteelement wird mit einer in distaler Richtung wirkenden axialen Kraft beispielsweise in Form eines Werkzeugs, beispielsweise mit Hilfe einer Stoss- oder Schiebeeinrichtung, relativ zu dem Karpulenhalter, insbesondere dem Karpulenhaltergehäuse verschoben derart dass, der Vorsprung des Halteelements aus der Ausnehmung des Karpulenhalters gelangt und eine Nocke des Halteelements in die Ausnehmung des Karpulenhalters rastet, um das Halteelement elastisch vorzuspannen. Dabei kann das Halteelement in axialer und radialer Richtung vorgespannt werden derart, dass die Karpule spielfrei mit dem Karpulenhalter verbunden ist, wobei ein Klemmelement des Halteelements einen proximalen Karpulenrand der Karpule hält oder umgreift. Die Karpule wird in dem Karpulenhalter mit Hilfe des Halteelements an der proximalen Seite gehalten.

Die Erfindung wird im Folgenden an mehreren Figuren beschrieben. Die hierbei offenbarten Merkmale bilden je einzeln und in Kombination die Erfindung vorteilhaft weiter. Es zeigen:
- Figur 1:: eine Explosionsansicht einer Injektionsvorrichtung zur Aufdosierung und zur Ausschüttung einer festgelegten Dosis eines fluiden Produkts mit einer ersten Ausführungsform eines Karpulenhalters
- Figur 2a:: eine Längsschnittansicht der ersten Ausführungsform des Karpulenhalters in einer Anfangsposition, bei welcher die Karpule im Karpulenhalten nicht aufgenommen ist
- Figur 2b:: eine Längsschnittansicht der ersten Ausführungsform des Karpulenhalters in einer ersten Position, bei welcher die Karpule im Karpulenhalter bewegbar aufgenommen ist
- Figur 2c:: eine Detailansicht der Figur 2b
- Figur 2d:: eine Längsschnittansicht der ersten Ausführungsform des Karpulenhalters in einer zweiten Position, bei welcher die Karpule spielfrei im Karpulenhalter aufgenommen ist
- Figur 2e:: eine Detailansicht der Figur 2d
- Figur 2f:: eine Längsschnittansicht der Injektionsvorrichtung mit der ersten Ausführungsform des mit der Karpule aufgenommenen und befestigten Karpulenhalters, wobei der Karpulenhalter mit dem Gehäuse der Injektionsvorrichtung zur Aufdosierung und zur Ausschüttung einer festgelegten Dosis eines fluiden Produkts axial fest verbunden ist
- Figur 3:: eine Explosionsansicht einer Injektionsvorrichtung zur Aufdosierung und zur Ausschüttung einer festgelegten Dosis eines fluiden Produkts mit einer zweiten Ausführungsform eines Karpulenhalters
- Figur 4a:: eine Längsschnittansicht der zweiten Ausführungsform des Karpulenhalters in einer Anfangsposition, bei welcher die Karpule im Karpulenhalten nicht aufgenommen ist
- Figur 4b:: eine Längsschnittansicht der zweiten Ausführungsform des Karpulenhalters in einer ersten Position, bei welcher die Karpule im Karpulenhalter bewegbar aufgenommen ist
- Figur 4c:: eine Detailansicht der Figur 4b
- Figur 4d:: eine Längsschnittansicht der zweiten Ausführungsform des Karpulenhalters in einer zweiten Position, bei welcher die Karpule spielfrei im Karpulenhalter aufgenommen ist
- Figur 4e:: eine Detailansicht der Figur 4d
- Figur 4f:: eine Längsschnittansicht der Injektionsvorrichtung mit der zweiten Ausführungsform des mit der Karpule aufgenommenen und befestigten Karpulenhalters, wobei der Karpulenhalter mit dem Gehäuse der Injektionsvorrichtung zur Aufdosierung und zur Ausschüttung einer festgelegten Dosis eines fluiden Produkts axial fest verbunden ist
- Figur 5:: eine Explosionsansicht einer Injektionsvorrichtung zur Aufdosierung und zur Ausschüttung einer festgelegten Dosis eines fluiden Produkts mit einer dritten Ausführungsform eines Karpulenhalters
- Figur 6a:: eine Längsschnittansicht der dritten Ausführungsform des Karpulenhalters in einer Anfangsposition, bei welcher die Karpule im Karpulenhalten nicht aufgenommen ist
- Figur 6b:: eine Längsschnittansicht der dritten Ausführungsform des Karpulenhalters in einer ersten Position, bei welcher die Karpule im Karpulenhalter bewegbar aufgenommen ist
- Figur 6c:: eine Detailansicht der Figur 6b
- Figur 6d:: eine Längsschnittansicht der dritten Ausführungsform des Karpulenhalters in einer zweiten Position, bei welcher die Karpule spielfrei im Karpulenhalter aufgenommen ist
- Figur 6e:: eine Detailansicht der Figur 6d
- Figur 6f:: eine Längsschnittansicht der Injektionsvorrichtung mit der dritten Ausführungsform des mit der Karpule aufgenommenen und befestigten Karpulenhalters, wobei der Karpulenhalter mit dem Gehäuse der Injektionsvorrichtung zur Aufdosierung und zur Ausschüttung einer festgelegten Dosis eines fluiden Produkts axial fest verbunden ist
- Figur 7:: eine Explosionsansicht einer Injektionsvorrichtung zur Aufdosierung und zur Ausschüttung einer festgelegten Dosis eines fluiden Produkts mit einer vierten Ausführungsform eines Karpulenhalters
- Figur 8a:: eine Längsschnittansicht der vierten Ausführungsform des Karpulenhalters in einer Anfangsposition, bei welcher die Karpule im Karpulenhalten nicht aufgenommen ist
- Figur 8b:: eine Längsschnittansicht der vierten Ausführungsform des Karpulenhalters in einer ersten Position, bei welcher die Karpule im Karpulenhalter bewegbar aufgenommen ist
- Figur 8c:: eine Detailansicht der Figur 8b
- Figur 8d:: eine Längsschnittansicht der vierten Ausführungsform des Karpulenhalters in einer zweiten Position, bei welcher die Karpule spielfrei im Karpulenhalter aufgenommen ist
- Figur 8e:: eine Detailansicht der Figur 8d
- Figur 8f:: eine Längsschnittansicht der Injektionsvorrichtung mit der vierten Ausführungsform des mit der Karpule aufgenommenen und befestigten Karpulenhalters, wobei der Karpulenhalter mit dem Gehäuse der Injektionsvorrichtung zur Aufdosierung und zur Ausschüttung einer festgelegten Dosis eines fluiden Produkts axial fest verbunden ist

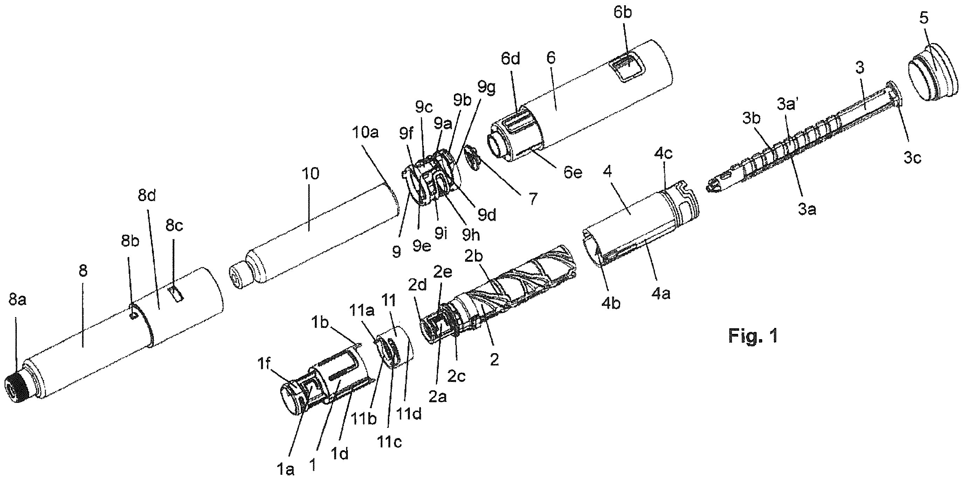

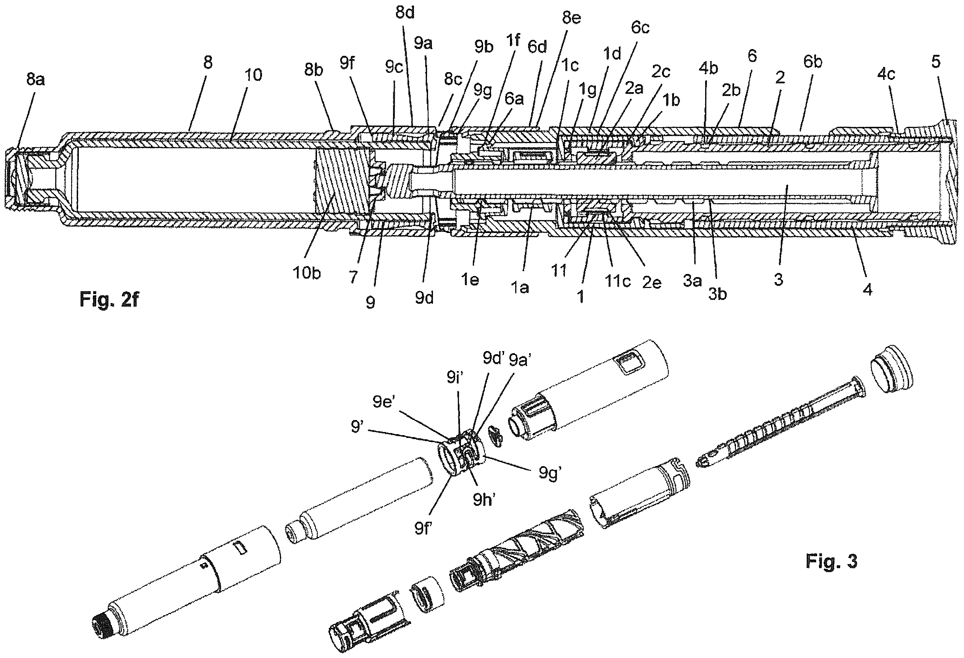

In der Figur 1 ist eine Explosionsansicht einer ersten Ausführungsform einer erfindungsgemässen Injektionsvorrichtung dargestellt. Die Injektionsvorrichtung umfasst einen Karpulenhalter (8), welcher eine Karpule (10) aufnehmen kann, wobei der Karpulenhalter (8) mit einem Gehäuse (6) axial fest verbunden werden kann. Diese Verbindung kann als Klebeverbindung oder als Schweissverbindung ausgebildet sein. Das Gehäuse (6) kann vorzugsweise ein Gehäuseführungssteg (6d) aufweisen, welcher in Eingriff mit einer an dem Karpulenhalter (8) angeordneten Karpulenhalterführungsnut (8e; beispielsweise in Figur 2f) sein kann. Ferner kann das Gehäuse (6) einen Gehäusesteg (6e), vorzugsweise mehrere Gehäusestege (6e) aufweisen. An dem distalen Ende des Karpulenhalters (8) ist eine Nadelverbindungselement (8a) vorgesehen, welches zur lösbaren Befestigung einer Injektionsnadel (nicht ersichtlich) vorgesehen ist. Die Injektionsvorrichtung umfasst ferner eine Verschlusskappe (nicht ersichtlich), welche über eine lösbare Verbindung mit dem Karpulenhalter (8) verbindbar ist. Dazu weist der Karpulenhalter (8) einen Karpulenhaltervorsprung (8b) auf, der in eine Ringnut (nicht ersichtlich) der Verschlusskappe (nicht ersichtlich) einrasten kann. Vorzugsweise kann die Ringnut (nicht ersichtlich) der Verschlusskappe (nicht ersichtlich) eine Vertiefung auf, sodass die Verschlusskappe (nicht ersichtlich) relativ zu dem Karpulenhalter (8) drehfest anordenbar ist. Der Karpulenhalter (8) umfasst ferner ein Karpulenhaltergehäuse (8d), in welchem eine Ausnehmung (8c) zur Aufnahme eines Vorsprungs (9a) und einer Nocke (9b) eines Halteelements (9) angeordnet ist. Das Halteelement (9) ist elastisch verformbar ausgebildet. Das Halteelement (9) umfasst ein Federelement (9c), an welchem der Vorsprung (9a) und ein Klemmelement (9d) vorgesehen sind. Das Halteelement (9) weist ein Ringelement (9e) auf, welches einen ersten (9f), einen zweiten Federsitz (9g), mehrere Bügel (9h) und mehrere Verbindungsstege (9i) umfasst, wobei der erste Federsitz (9f) über die mehreren Bügel (9h) und über die mehreren Verbindungsstege (9i) mit dem zweiten Federsitz (9g) verbunden ist. Ferner umfasst die Injektionsvorrichtung eine Führungshülse (1). Die Führungshülse (1) ist mit Gehäuse (6) über einen an der Führungshülse (1) vorgesehenen Führungshülsensteg (1d) und an dem Gehäuse (6) angebrachte Nut (6c; beispielsweise in Figur 2f) drehfest und über einen nach innen ragenden Führungshülsenvorsprung (1f) der Führungshülse (1) und einen an dem Gehäuse (6) angebrachten Gehäusehaltearm (6a; beispielsweise in Figur 2f) axial fest verbunden. Die Führungshülse (1) weist ein Eingriffselement (1a) auf, welches mit der Zahnung der Gewindestange (3) zusammenwirken kann. Die Injektionsvorrichtung umfasst ferner eine Rotationshülse (2), die axial fest und drehbar mit der Führungshülse (1) verbunden ist. Dazu weist die Rotationshülse (2) einen Rotationshülsenringsteg (2c) auf, der an dessen proximalen Seite axial an den Führungshülsenhaltearm (1b), vorzugsweise an zwei Führungshülsenhaitearme (1b) anschlagen kann, wobei ferner die Rotationshülsenkante (2d) einen axialen Anschlagkontakt mit einer Führungshülsenstimseite (1c, beispielsweise in Figur 2f) der Führungshülse (1) bilden kann. Die Rotationshülse (2) umfasst ebenfalls ein Eingriffselement (2a), welches mit der Zahnung der Gewindestange (3) zusammen wirken kann. Die Zahnung der Gewindestange (3) umfasst einen Zahn (3a), vorzugsweise mehrere Zähne, besonders bevorzugt vier Zähne. Ferner weist die Gewindestange (3) ein Aussengewinde (3b) auf, welches mit einem Innengewinde (1e, beispielsweise in Figur 2f) der Führungshülse (1) eine Gewindeverbindung bildet. Der Eingriffsmechanismus der Eingriffselemente (2a, 1a) der Rotationshülse (2) und der Führungshülse (1) in die Zahnung der Gewindestange (3) ist als Ratschenmechanismus ausgebildet. Die Eingriffselemente (2a, 1a) der Rotationshülse (2) und der Führungshülse (1) wirken mit der Zahnung der Gewindestange (3) derart zusammen, dass beim Aufdosieren einer festgelegten Dosis und beim Ausschütten einer festgelegten Dosis ein Klick-Geräusch erzeugt wird. Um ein Einstellen einer weiteren Dosis zu verhindern, wenn die letzte Dosis ausgeschüttet worden ist, weist die Rotationshülse (2) einen Rotationshülsenanschlag (nicht ersichtlich) auf, der in Anschlagkontakt mit einem an der Gewindestange (3) vorgesehenen Gewindestangenanschlag (3c) kommen kann. Die Injektionsvorrichtung umfasst ferner eine Dosierhülse (4). Die Dosierhülse (4) ist über einen Dosierhülsensteg (4a) mit einer Führungshülsennut (nicht ersichtlich) der Führungshülse (1) relativ zu dem Gehäuse (6) der Injektionsvorrichtung drehfest und axial bewegbar gelagert. Der Benutzer kann die Dosierhülse (4) mit Hilfe eines am proximalen Ende der Dosierhülse (1) angeordneten Dosierknopfs (5) angebracht sein, der über eine Dosierknopfsteg (nicht ersichtlich) mit einem Dosierhülsenringnut (4c) der Dosierhülse (4) axial fest verbunden ist, axial hin und her bewegen. Zur Anzeige der einzelnen Dosierbewegungen weist die Dosierhülse (1) vorzugweise eine Anzeige (nicht ersichtlich) auf. Die Anzeige (nicht ersichtlich) der Dosierhülse (1) kann durch ein an dem Gehäuse (6) vorgesehenes Gehäusefenster (6b) ersichtlich sein, wobei für den Benutzer ersichtlich sein kann, in welcher Position die Injektionsvorrichtung ist. Die Dosierhülse (4) ist über eine Gewindeverbindung mit der Rotationshülse (2) verbunden. Die Innenseite der Dosierhülse (4) umfasst ein Dosierhülsengewinde (4b), welches in Eingriff mit dem Rotationshülsengewinde (2b) an der Aussenseite der Rotationshülse (2) ist. Die Injektionsvorrichtung umfasst ferner ein Verriegelungsring (11), der an dessen distalen Ende einen Ratschenzahn (11a) aufweist, der über einen Riegelarm (11b) des Verriegelungsrings (11) mit der Führungshülse (1) verbunden ist. Der Ratschenzahn (11a) des Verriegelungsrings (11) kann in eine Ratschenverzahnung (1g, beispielsweise in Figur 2f) der Führungshülse (1) eingreifen, wobei der Ratschenzahn (11a) des Verriegelungsrings (11) und die Ratschenverzahnung (1g, beispielsweise in Figur 2f) der Führungshülse (1) derart ausgebildet sind, dass der Ratschenzahn (11a) relativ zu der Ratschenverzahung (1g, beispielsweise in Figur 2f) der Führungshülse (1) in eine erste Drehrichtung über die Ratschenverzahnung (1g, beispielsweise in Figur 2f) der Führungshülse (1) gleiten kann und in eine der ersten Drehrichtung entgegengesetzte Drehrichtung, nämlich in eine zweite Drehrichtung eine relative Drehung zwischen dem Verriegelungsring (11) und der Führungshülse (1) verhindert werden kann. Der Verriegelungsring kann nicht relativ zu dem Gehäuse (6) oder der Führungshülse (1) in axialer Richtung verschoben werden. Ferner umfasst der Verriegelungsring (11) an dessen Mantelinnenfläche eine Kupplungsverzahung (11c), welche mit einer an der Mantelaussenfläche des Eingriffselements (2a) der Rotationshülse (1) angeordneten Gegenkupplungsverzahnung (2e) in Eingriff gelangen kann. Am proximalen Ende des Verriegelungsrings (11) ist eine Anschlagfläche (11d) angeordnet, die in Anschlagkontakt mit einer distalen Seite des Rotationshülsenringstegs (2c) gelangen kann.

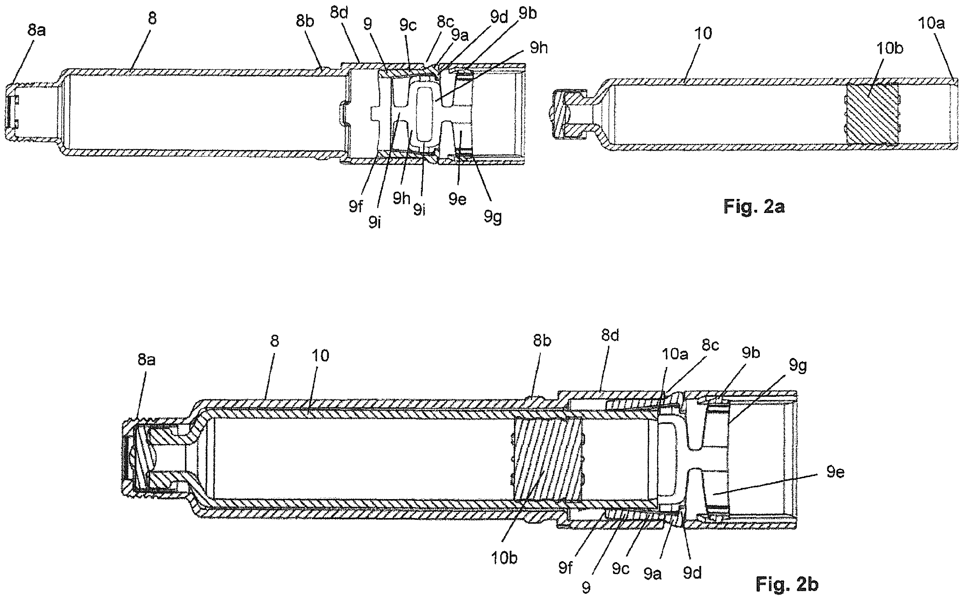

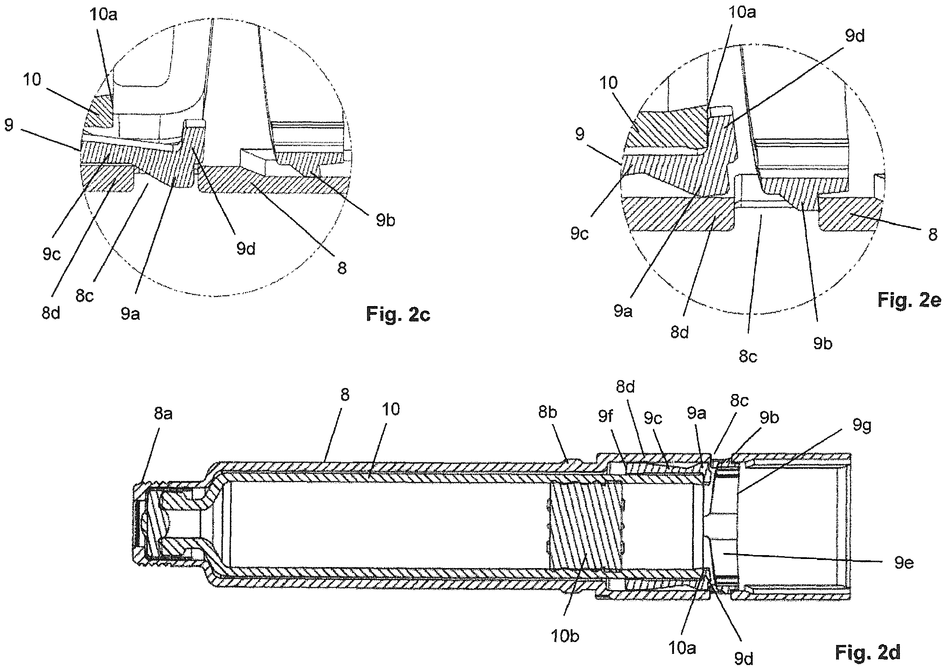

In der Figur 2a ist eine Längsschnittansicht der ersten Ausführungsform des Karpulenhalters in einer Anfangsposition, bei welcher die Karpule im Karpulenhalter nicht aufgenommen ist, dargestellt. Das Halteelement (9) ist in dem Karpulenhalter (8) eingesetzt. Wie in der Figur 2c ersichtlich, ragt der Vorsprung (9a) des Halteelements (9) in die Ausnehmung (8c) des Karpulenhaltergehäuses (8d) des Karpulenhalters (8). Die Nocke (9b) des Halteelements (9) liegt radial elastisch vorgespannt an der Mantelinnenfläche des Karpulenhalters (8) an. Das Halteelement (9) ist in axialer Richtung elastisch entspannt in dem Karpulenhalter (8) gelagert. Die Bügel (9h) sind über Verbindungsstege (9i) miteinander verbunden und über Verbindungsstege (9i) an dem ersten (9f) beziehungsweise an dem zweiten Federsitz (9g) befestigt. Das Halteelement (9) ist derart in den Karpulenhalter eingesetzt, dass eine Karpule (10) in den Karpulenhalter (8), insbesondere in das Karpulenhaltergehäuse (8d) des Karpulenhalters (8) eingefügt werden kann. Die Karpule (10) wird durch eine proximale Öffnung des Karpulenhaltergehäuses (8d) des Karpulenhalters (8) in den Karpulenhalter (8) geschoben. Wie in der Figur 2b ersichtlich, kann vorzugsweise das distale Ende der Karpule (10) in axialen Anschlagkontakt mit dem distalen Ende des Karpulenhaltergehäuses (8d) des Karpulenhalters (8) gelangen. Das Halteelement (9) befindet sich in der ersten Position. Das Halteelement (9) ist in einem Ringspalt zwischen dem Karpulenhalter (8) und der Karpule (10) angeordnet. Das Klemmelement (9d) des Federelements (9c) des Halteelements (9) ragt in proximaler Richtung über den proximalen Karpulenrand (10a) der Karpule (10). Um die Karpule (10) in dem Karpulenhalter (8) spielfrei zu halten, kann eine axiale Kraft in distaler Richtung auf den zweiten Federsitz (9g) derart wirken, dass das Halteelement (8) elastisch vorgespannt und relativ zu dem Karpulenhaltergehäuse (8d) des Karpulenhalters (8) in axialer Richtung verschoben wird, Wie in der Figur 2d und in der Figur 2e ersichtlich ist, gelangt der Vorsprung (9a) des Halteelements (9) ausser Eingriff mit der Ausnehmung (8c) des Karpulenhalters (8), wobei die Nocke (9b) des Halteelements (9) in Eingriff mit der Ausnehmung (8c) des Karpulenhalters (8) kommt. Das Federelement (9c) des Halteelements (9) wird dabei radial nach innen vorgespannt, so dass das Klemmelement (9d) den proximalen Karpulenrand (10a) der Karpule (10) hält oder umgreift. Die Karpule (10) ist am distalen Ende an oder in dem Karpulenhalter (8) und am proximalen Ende an dem Halteelement (9), insbesondere an dem Klemmelement (9d) des Haltelements (9) spielfrei abgestützt, Wie in der Figur 2f ersichtlich, kann der mit Karpule (10) eingesetzte Karpulenhalter (8) nun mit dem Gehäuse (6) der Injektionsvorrichtung axial fest verbunden werden, Der Gehäuseführungssteg (6d) des Gehäuses (6) ist im Eingriff mit der Karpulenhalterführungsnut (8e) des Karpulenhalters (8). Der Karpulenhalter (8) kann mit dem Gehäuse (6) der Injektionsvorrichtung verklebt oder verschweisst werden. Es können auch andere Befestigungen, beispielsweise mit anderen Befestigungsmittel zwischen dem Karpulenhalter (8) und dem Gehäuse (6) vorgesehen sein. Vorzugsweise kann zur besseren Befestigung des Karpulenhalters (8) an oder in das Gehäuse (6) der Injektionsvorrichtung zwischen den in axialer Richtung erstreckenden Gehäusestegen (6e) des Gehäuses (6) der Injektionsvorrichtung Klebstoff oder ein alternatives Befestigungsmittel aufgetragen werden. Alternativ kann der Karpulenhalter (8) einen Karpulenhaltersteg, vorzugsweise mehrere in axialer Richtung ersteckenden Karpulenhalterstege aufweisen, zwischen welchen Klebstoff oder ein alternatives Befestigungsmittel aufgetragen wird. Alternativ kann an dem Karpulenhalter (8) Karpulenhalterstege und an dem Gehäuse (6) der Injektionsvorrichtung Gehäusestege (6e) vorgesehen sein.

In der Figur 2f ist eine Längsschnittansicht der ersten Ausführungsform der Injektionsvorrichtung in einer Anfangsposition einer Dosierhülse (4) dargestellt. Die im Gehäuse (6) axial bewegbar und drehfest gelagerte Dosierhülse (4) ist in dem Injektionsgerät eingeschoben, wobei die an der Dosierhülse (4) angebrachte Anzeige (nicht ersichtlich) durch das Gehäusefenster (6b) ersichtlich ist und dem Benutzer anzeigt, dass die Injektionsvorrichtung in der Anfangsposition ist. Die Dosierhülse (4) ist über das Dosierhülsengewinde (4b) mit dem Rotationshülsengewinde (2b) der Rotationshülse (2) in Gewindeeingriff. Ein radial nach aussen ragender Vorsprung (nicht ersichtlich) der Rotationshülse (2) befindet sich in Anschlagkontakt, insbesondere in Rotationsanschlagkontakt mit einem ersten radial nach innen ragenden Vorsprung (nicht ersichtlich) der Führungshülse (1). Ein an dem Eingriffselement (2a) der Rotationshülse (2) angebrachter Kopf (nicht ersichtlich) ist im Eingriff mit der Zahnung der Gewindestange (3). Dazu greift der Kopf (nicht ersichtlich) des Eingriffselements (2a) der Rotationshülse (2) zwischen zwei benachbarte Zähne (3a) in eine Zahnlücke (nicht ersichtlich) der Gewindestange (3), nämlich zwischen einer steilen Flanke (nicht ersichtlich) eines Zahns (3a) und einer flachen Flanke (nicht ersichtlich) eines benachbarten Zahns (3a) der Gewindestange (3). Ferner ist ein an dem Eingriffselement (1a) der Führungshülse (1) vorgesehener Kopf (nicht ersichtlich) ebenfalls im Eingriff mit der Zahnung der Gewindestange (3). Dazu greift der Kopf (nicht ersichtlich) des Eingriffselements (1a) der Führungshülse (1) ebenfalls zwischen zwei benachbarten Zähnen (3a) in eine Zahnlücke (nicht ersichtlich) der Gewindestange (3). Der Kopf (nicht ersichtlich) des Eingriffselements (1a) der Führungshülse befindet sich zwischen einer steilen Flanke (nicht ersichtlich) eines Zahns (3a) und einer flachen Flanke (nicht ersichtlich) eines benachbarten Zahns (3a) der Gewindestange (3).

Um eine Dosis einzustellen oder aufzuziehen, zieht der Benutzer den Dosierknopf (5) in die proximale Richtung. Die Dosierhülse (4) wird dabei axial in die proximale Richtung verschoben. Aufgrund des Gewindeeingriffs zwischen der Dosierhülse (4) und der Rotationshülse (2), dreht die Rotationshülse (2) in die erste Drehrichtung bis der Vorsprung (nicht ersichtlich) der Rotationshülse (2) in Anschlagkontakt mit einem zweiten radial nach innen ragenden Vorsprung (nicht ersichtlich) der Führungshülse (1) in Anschlagkontakt. Die flache Flanke (nicht ersichtlich) des Kopfs (nicht ersichtlich) des Eingriffselements (2a) der Rotationshülse (2) gleitet dabei über die flache Flanke (3a') des Zahns (3a) der Gewindestange (3) und gelangt dabei in eine benachbarte Zahnlücke (nicht ersichtlich). Die steile Flanke (nicht ersichtlich) des Kopfs (nicht ersichtlich) des Eingriffselements (1a) der Führungshülse (1) in Anschlagkontakt mit der steilen Flanke (nicht ersichtlich) des Zahns (3a) der Gewindestange (3), so dass die Gewindestange (3) rotativ fest gehalten wird. Die an der Dosierhülse (4) angebrachte Anzeige (nicht ersichtlich) zeigt dem Benutzer durch das Gehäusefenster (6b) an, dass die Injektionsvorrichtung in der aufgezogenen Position ist.

Bei der Einstellung oder beim Aufziehen einer Dosis wird das Eingriffselement (2a) der Rotationshülse (2) radial ausgelenkt. Dabei gelangt die Gegenkupplungsverzahnung (2e) der Rotationshülse (2) in Eingriff mit der Kupplungsverzahnung (11c) des Verriegelungsrings (11). Durch die Kuppelung zwischen der Rotationshülse (2) und des Verriegelungsrings (11) wird der Verriegelungsring (11) und die Rotationshülse (2) in die erste Drehrichtung relativ zu dem Gehäuse (6) oder der Führungshülse (1) gedreht, Der Ratschenzahn (11a) des Verriegelungsrings (11) gleitet über die Ratschenverzahnung (1g) der Führungshülse (1). Der Ratschenzahn (11a) des Verriegelungsrings (11) kann nur in die erste Drehrichtung relativ zu der Führungshülse (1) über die Ratschenverzahnung (1g) der Führungshülse (1) gleiten, während eine Relativdrehung in die zweite Drehrichtung verhindert wird. Das Eingriffselements (2a) der Rotationshülse (2) ist so lange radial ausgelenkt, bis der Kopf (nicht ersichtlich) des Eingriffselements in die Zahnlücke (3a") der Gewindestange (3) gelangt, beziehungsweise bis eine festgelegte Dosis vollständig eingestellt oder aufgezogen ist. Somit kann verhindert werden, dass eine unvollständig eingestellte oder aufgezogene Dosis ausgeschüttet werden kann.

Die Aufziehbewegung der Dosierhülse (4) und die Ausschüttbewegung der Gewindestange (3) sind übersetzt. Die Gewindesteigung der Gewindeverbindung (4b, 2b) zwischen der Dosierhülse (4) und der Rotationshülse (2) ist grösser als die Gewindesteigung der Gewindeverbindung (3b, 1e) zwischen der Gewindestange (3) und der Führungshülse (1).

Um eine festgelegte Dosis auszuschütten, drückt der Benutzer den Dosierknopf (5) in die distale Richtung, wobei die Dosierhülse (4) in die distale Richtung axial verschoben wird. Die Rotationshülse (2) dreht in die der ersten Drehrichtung entgegengesetzte Drehrichtung, nämlich in die zweite Drehrichtung bis der Vorsprung (nicht ersichtlich) der Rotationshülse (2) in Anschlagkontakt mit dem ersten radial nach innen ragenden Vorsprung (nicht ersichtlich) der Führungshülse (1) in Anschlagkontakt gelangt. Die steile Flanke (nicht ersichtlich) des Kopfs (nicht ersichtlich) des Eingriffselements (2a) der Rotationshülse (2) ist in Anschlagkontakt mit der steilen Flanke (nicht ersichtlich) des Zahns (3a) der Gewindestange (3) und überträgt ein Drehmoment auf die Gewindestange (3). Die Gewindestange (3) schraubt sich dadurch aufgrund der Gewindeverbindung zwischen dem Innengewinde (1e) der Führungshülse (1) und dem Aussengewinde (3b) der Gewindestange (3) in die distale Richtung. Dabei gleitet der Kopf (nicht ersichtlich) des Eingriffselements (2a) der Rotationshülse (2) axial entlang der Zahnlücke (nicht ersichtlich) der Gewindestange (3). Die Kuppelungsverzahnung (11c) des Verriegelungsrings (11) ist von der Gegenkupplungsverzahnung (2e) der Rotationshülse (2) entkuppelt, so dass die Rotationshülse (2) relativ zu dem Verriegelungsring (11) in die zweite Drehrichtung drehen kann. Der Ratschenzahn (11c) des Verriegelungsrings (11) ragt in die Ratschenzahnung (1g) der Führungshülse (1). Die flache Flanke des Kopfs (nicht ersichtlich) der Führungshülse (1) gleitet über die flache Flanke des Zahns (3a') der Gewindestange. Dabei wird über einen an der Gewindestange (3) axial fest angebrachten Flansch (7) ein von einer Karpule (10) aufgenommenen Stopfen (10a) in die distale Richtung gestossen. Der Stopfen (10b) kann das fluide Produkt über eine an dem Karpulenhalter befestigten Injektionsnadel (nicht ersichtlich) herauspressen.

Nachdem die letzte Dosis aus der Injektionsvorrichtung ausgeschüttet worden ist, sind zwei Rotationshülsenanschläge (nicht ersichtlich) der Rotationshülse (2) in Anschlagkontakt mit den entsprechenden zwei Gewindestangenanschläge (3c) der Gewindestange (3). Es kann somit keine weitere Dosis über die Dosierhülse (4) aufgezogen werden, da eine Rotation der Rotationshülse (2) blockiert wird.

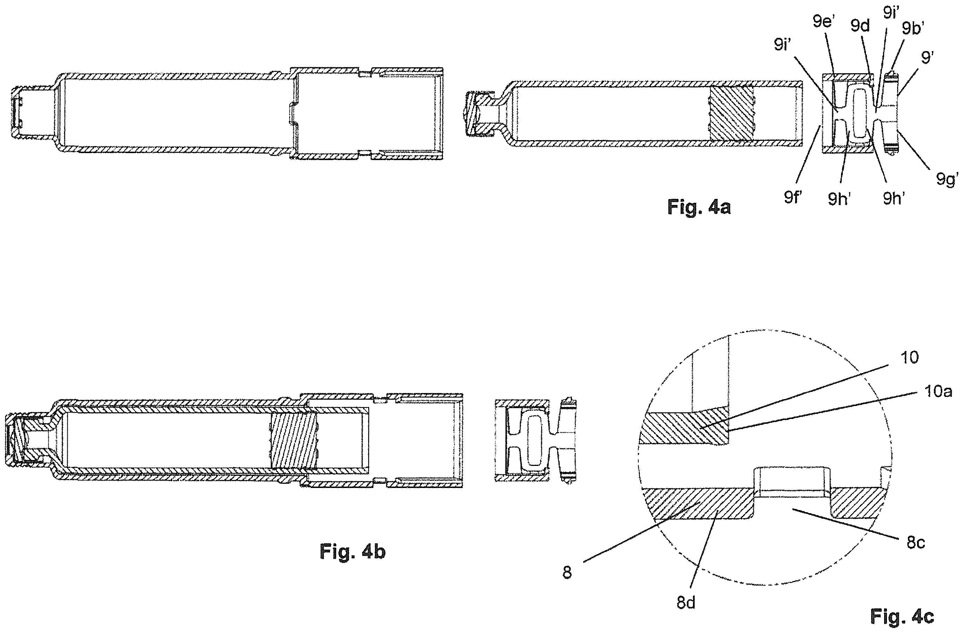

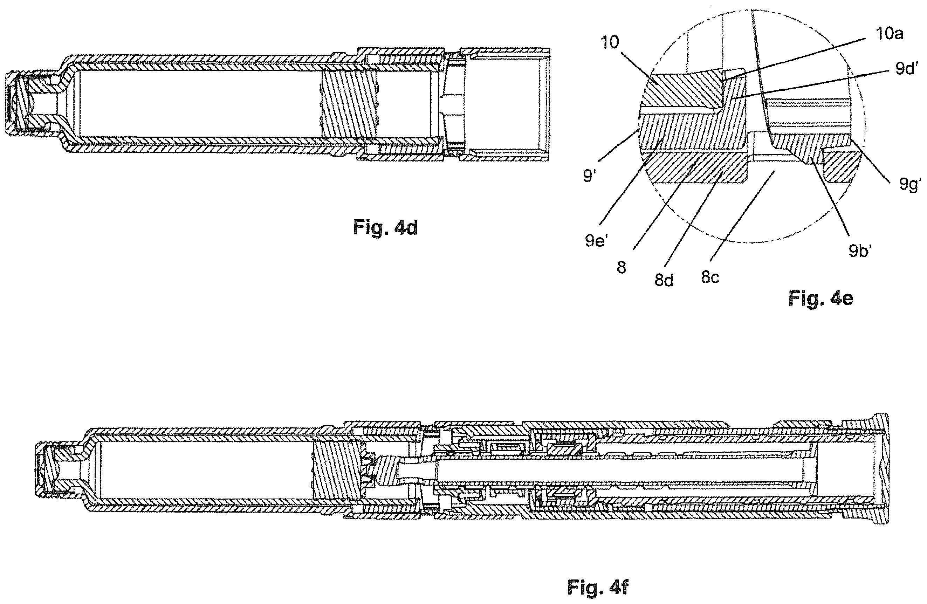

In der Figur 3 ist eine Explosionsansicht einer zweiten Ausführungsform einer erfindungsgemässen Injektionsvorrichtung dargestellt. Die Injektionsvorrichtung unterscheidet sich von der Injektionsvorrichtung der ersten Ausführungsform im Wesentlichen nur in Bezug auf das Halteelement (9'). Wie in der Figur 4a gezeigt, umfasst das Halteelement (9') einen ersten (9f') und einen zweiten Federsitz (9g') und ein Ringelement (9e'), wobei der erste (9f) und der zweite Federsitz (9g') über mehrere Bügel (9h') und über mehrere Verbindungsstege (9i') miteinander verbunden sind, und das Ringelement (9e') die mehreren Bügel (9h') und die mehreren Verbindungsstege (9i') in Umfangsrichtung umfasst. An dem zweiten Federsitz (9g') ist eine nach aussen ragende Nocke (9b') angeordnet. An dem Ringelement (9e') ist ein nach innen ragendes Klemmelement (9d') vorgesehen. Wie in der Figur 4b und in der Figur 4c gezeigt, wird zuerst die Karpule (10) in den Karpulenhalter (8) eingesetzt, so dass Karpule (10) und der Karpulenhalter (8) in am distalen Ende in axialen Anschlagkontakt sind. Danach wird das Halteelement (9') in der Karpulenhalter (8) eingebracht derart, dass das Halteelement (9') in einem Ringspalt zwischen dem Karpulenhalter (8) und der Karpule (10) angeordnet ist. Das Halteelement (9') wird mit Hilfe einer axialen Kraft, welche auf den zweiten Federsitz (9g') in distaler Richtung wirkt, relativ zu dem Karpulenhaltergehäuse (8d) in das Karpulenhaltergehäuse (8d) geschoben. Wie in der Figur 4d und in der Figur 4e gezeigt, rastet die Nocke (9b') des Halteelements (9') in die Ausnehmung (8d) des Karpulenhalters (8) ein. Ferner hält oder umgreift das Klemmelement (9d') des Halteelement (9') den proximalen Karpulenrand (10a) der Karpule (10). Das Halteelement (9') wird dabei in distaler Richtung vorgespannt mit Hilfe des Eingriffs zwischen der Nocke (9b') und Ausnehmung (8c) gehalten. Die Karpule (10) ist mit Hilfe des Halteelements (9') in dem Karpulenhalter (8) spielfrei fixiert. Wie in der Figur 4f ersichtlich, kann der mit der Karpule (10) verbundene Karpulenhalter (8) nun mit dem Gehäuse (6) der Injektionsvorrichtung axial fest verbunden werden. Der Karpulenhalter (8) kann mit dem Gehäuse (6) der Injektionsvorrichtung verklebt oder verschweisst werden. Es können auch andere Befestigungen, beispielsweise mit anderen Befestigungsmittel zwischen dem Karpulenhalter (8) und dem Gehäuse (6) vorgesehen sein. Vorzugsweise kann zur besseren Befestigung des Karpulenhalters (8) an oder in das Gehäuse (6) der Injektionsvorrichtung zwischen den in axialer Richtung erstreckenden Gehäusestegen (6e) des Gehäuses (6) der Injektionsvorrichtung Klebstoff oder ein alternatives Befestigungsmittel aufgetragen werden. Alternativ kann der Karpulenhalter (8) einen Karpulenhaltersteg, vorzugsweise mehrere in axialer Richtung ersteckenden Karpulenhalterstege aufweisen, zwischen welchen Klebstoff oder ein alternatives Befestigungsmittel aufgetragen wird. Alternativ kann an dem Karpulenhalter (8) Karpulenhalterstege und an dem Gehäuse (6) der Injektionsvorrichtung Gehäusestege (6e) vorgesehen sein,

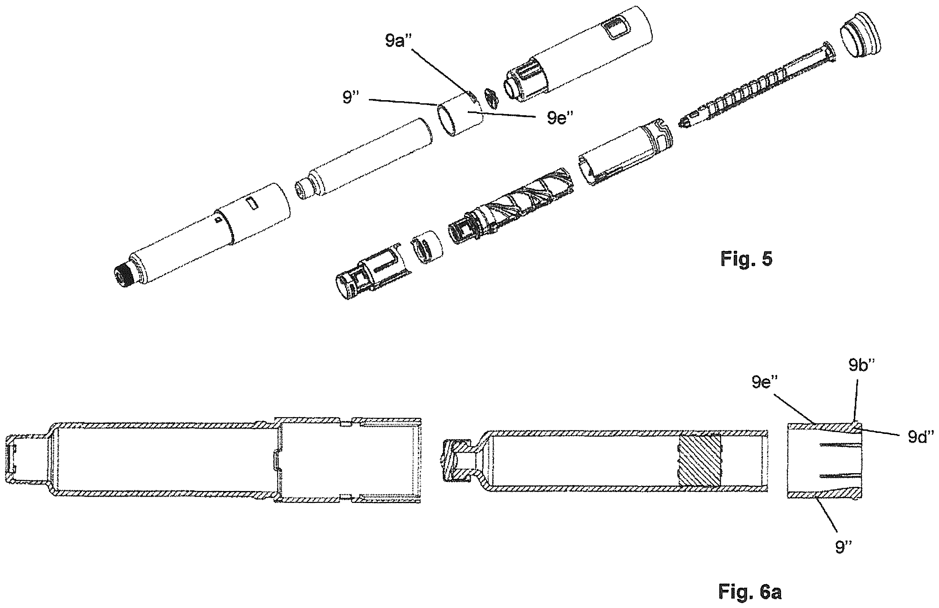

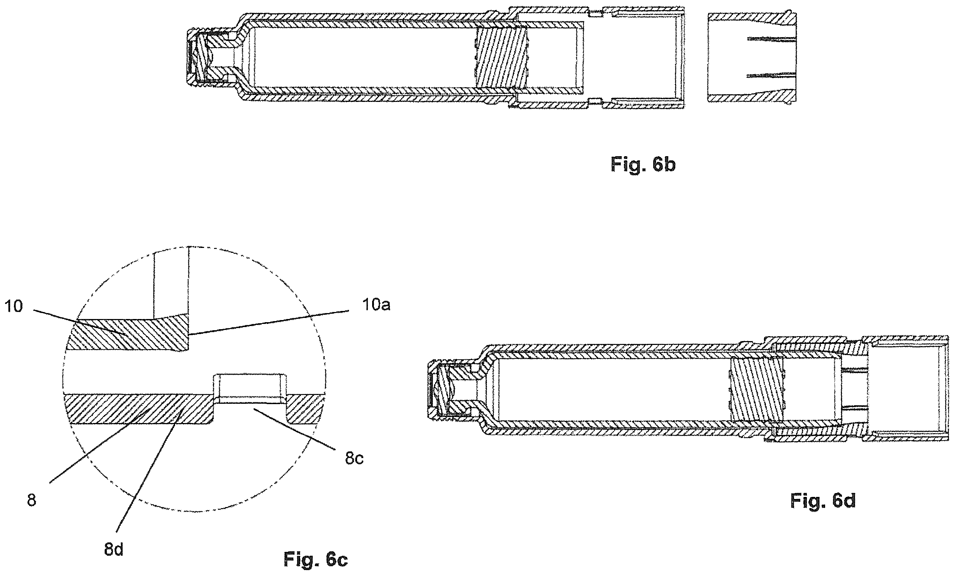

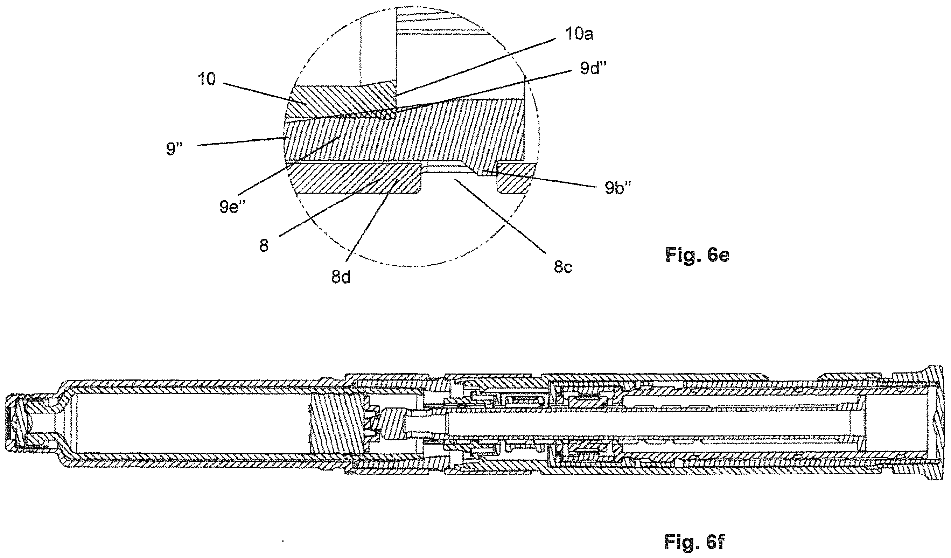

In der Figur 5 ist eine Explosionsansicht einer dritten Ausführungsform einer erfindungsgemässen Injektionsvorrichtung dargestellt. Die Injektionsvorrichtung unterscheidet sich von der Injektionsvorrichtung der ersten Ausführungsform im Wesentlichen nur in Bezug auf das Halteelement (9"). Wie in der Figur 6a ersichtlich ist, ist das Halteelement (9") als zylinderförmiges Ringelement (9e") ausgebildet. Wie in der Figur 6b und der Figur 6c gezeigt ist, wird zuerst die Karpule (10) in den Karpulenhalter (8) gesetzt, wobei der Karpulenhalter (8) eine Ausnehmung (8c) aufweist. Die Karpule (10) und der Karpulenhalter (8) kommen dabei am distalen Ende in axialen Anschlagkontakt. Danach kann das Halteelement (9") in ein mit der Karpule (10) aufgenommenen Karpulenhalter (8) in einen Ringspalt zwischen der Karpule (10) und dem Karpulenhalter (8) in distaler Richtung eingesetzt werden. Dazu kann eine axiale Kraft auf das proximale Ende des Ringelements (9e") des Halteelements (9") in distaler Richtung wirken. Wie in der Figur 6d und in der Figur 6e ersichtlich ist, kann eine nach aussen ragende Nocke (9b") des Ringelements (9e") dabei in die Ausnehmung (8c) des Karpulenhaltergehäuses (8d) des Karpulenhalters (8) gelangen. Ein nach innen ragendes Klemmelement (9d") des Ringelements (9e") des Halteelements (9") klemmt den proximalen Karpulenrand (10a) der Karpule (10) ein. Durch diese Klemmpassung beziehungsweise durch diesen Klemmsitz ist die Karpule (10) in dem Karpulenhalter (8) axial spielfrei aufgenommen. Die Karpule (10) ist am distalen Ende an oder in dem Karpulenhalter (8) und am proximalen Ende am Halteelement (9"), insbesondere am Klemmelement (9d") abgestützt. Wie in der Figur 6f ersichtlich, kann der mit der Karpule (10) verbundene Karpulenhalter (8) nun mit dem Gehäuse (6) der Injektionsvorrichtung axial fest verbunden werden. Der Karpulenhalter (8) kann mit dem Gehäuse (6) der Injektionsvorrichtung verklebt oder verschweisst werden. Es können auch andere Befestigungen, beispielsweise mit anderen Befestigungsmittel zwischen dem Karpulenhalter (8) und dem Gehäuse (6) vorgesehen sein. Vorzugsweise kann zur besseren Befestigung des Karpulenhalters (8) an oder in das Gehäuse (6) der Injektionsvorrichtung zwischen den in axialer Richtung erstreckenden Gehäusestegen (6e) des Gehäuses (6) der Injektionsvorrichtung Klebstoff oder ein alternatives Befestigungsmittel aufgetragen werden. Alternativ kann der Karpulenhalter (8) einen Karpulenhaltersteg, vorzugsweise mehrere in axialer Richtung ersteckenden Karpulenhalterstege aufweisen, zwischen welchen Klebstoff oder ein alternatives Befestigungsmittel aufgetragen wird. Alternativ kann an dem Karpulenhalter (8) Karpulenhalterstege und an dem Gehäuse (6) der Injektionsvorrichtung Gehäusestege (6e) vorgesehen sein.

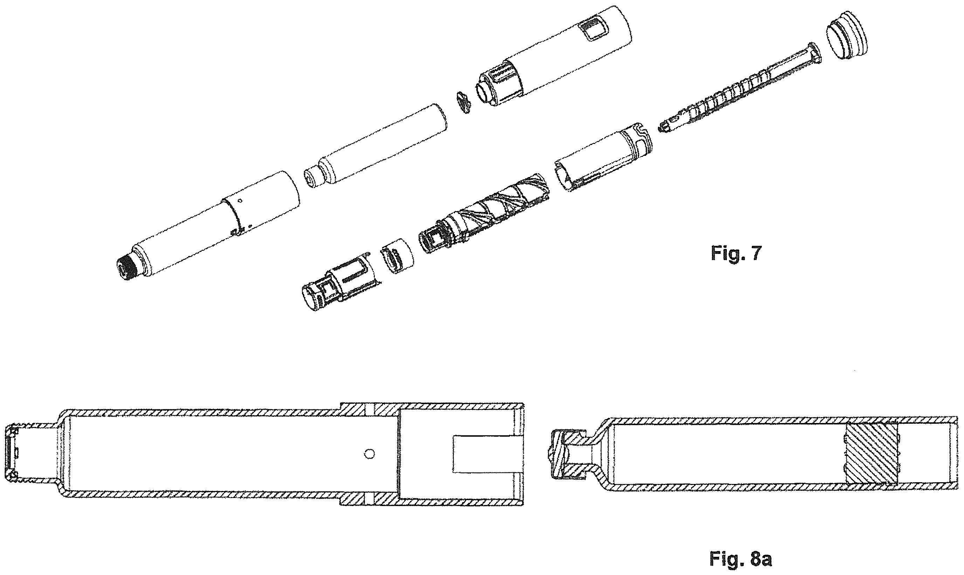

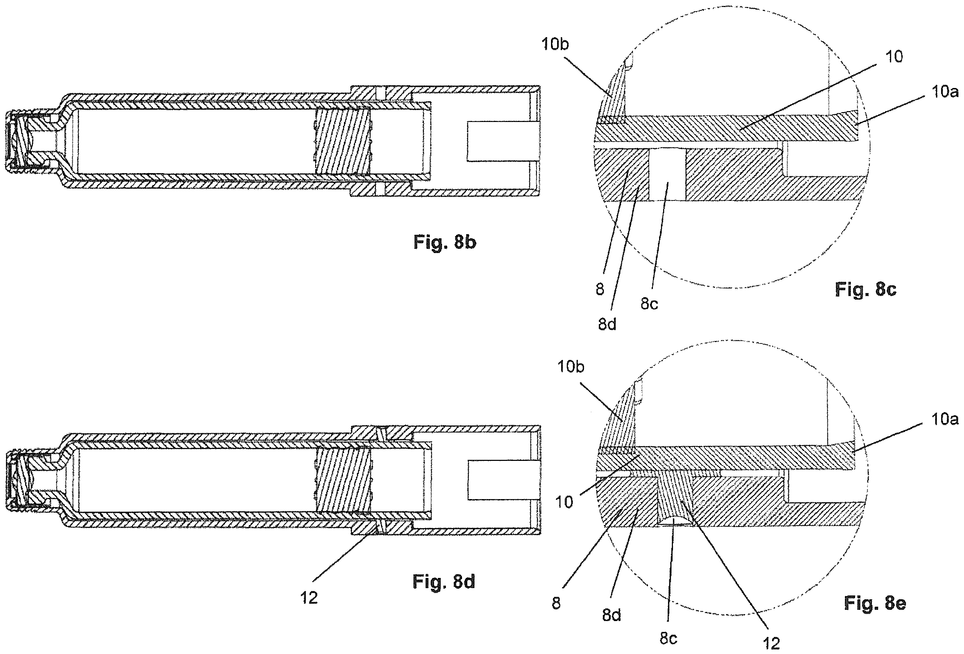

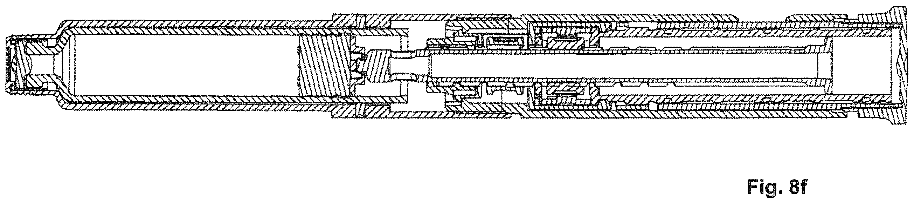

In der Figur 7 ist eine Explosionsansicht einer vierten Ausführungsform einer erfindungsgemässen Injektionsvorrichtung dargestellt. Die Injektionsvorrichtung unterscheidet sich von der Injektionsvorrichtung der ersten Ausführungsform im Wesentlichen nur in Bezug auf das Fehlen des Halteelements (9, 9', 9"). Wie in der Figur 8a ersichtlich ist, wird die Karpule (10) in den Karpulenhalter (8) eingefügt, bis die Karpule (10) in distalem Anschlagkontakt mit dem Karpulenhalter (8) gelangt, wie in der Figur 8b gezeigt wird. Wie in der Figur 8c ersichtlich ist, umfasst der Karpulenhalter (8) in dessen Karpulenhaltergehäuse (8d) eine Ausnehmung (8c). Wie in der Figur 8d und in der Figur 8e ersichtlich ist, wird zur Fixierung der Karpule (10) in dem Karpulenhalter (8) durch die Ausnehmung (8c) des Karpulenhaltergehäuses (8d) ein Klebstoff (12) oder ein anderes Befestigungsmittel eingefügt derart, dass die Karpule (10) in dem Karpulenhalter axial spielfrei gehalten wird. Der Klebstoff (12) oder das Befestigungsmittel kann in Form eines oder mehreren Tropfens aufgetragen werden. Alternativ kann zwischen der Karpule (10) und dem Karpulenhalter (8) eine Schweissverbindung vorgesehen sein. Die Karpule (10) wird am distalen Ende an oder in dem Karpulenhalter (8) gehalten und am proximalen Ende mit dem Karpulenhalter befestigt, Die Befestigung der Karpule (10) an dem Karpulenhalter (8) kann vorzugsweise zwischen dem Karpulenhaltergehäuse (8d) und der Seitenwand der Karpule (10) erfolgen. Alternativ kann die Befestigung an dem proximalen Karpulenrand (10a) der Karpule (10) und dem Karpulenhaltergehäuse (8d) des Karpulenhalters (8) erfolgen. Wie in der Figur 8f ersichtlich, kann der mit der Karpule (10) verbundene Karpulenhalter (8) nun mit dem Gehäuse (6) der Injektionsvorrichtung axial fest verbunden werden. Der Karpulenhalter (8) kann mit dem Gehäuse (6) der Injektionsvorrichtung verklebt oder verschweisst werden. Es können auch andere Befestigungen, beispielsweise mit anderen Befestigungsmittel zwischen dem Karpulenhalter (8) und dem Gehäuse (6) vorgesehen sein. Vorzugsweise kann zur besseren Befestigung des Karpulenhalters (8) an oder in das Gehäuse (6) der Injektionsvorrichtung zwischen den in axialer Richtung erstreckenden Gehäusestegen (6e) des Gehäuses (6) der Injektionsvorrichtung Klebstoff oder ein alternatives Befestigungsmittel aufgetragen werden. Alternativ kann der Karpulenhalter (8) einen Karpulenhaltersteg, vorzugsweise mehrere in axialer Richtung ersteckenden Karpulenhalterstege aufweisen, zwischen welchen Klebstoff oder ein alternatives Befestigungsmittel aufgetragen wird. Alternativ kann an dem Karpulenhalter (8) Karpulenhalterstege und an dem Gehäuse (6) der Injektionsvorrichtung Gehäusestege (6e) vorgesehen sein.

### Bezugszeichen:

- 1: Führungshülse
1a Eingriffselement
1b Führungshülsenhaltearm
1c Führungshülsenstirnseite
1d Führungshülsensteg
1e Innengewinde
1f Führungshülsenvorsprung
1g Ratschenverzahnung
- 2: Rotationshülse
2a Eingriffselement
2b Rotationshülsengewinde
2c Rotationshülsenringsteg
2d Rotationshülsenkante
2e Gegenkuppelungsverzahnung
- 3: Gewindestange
3a Zahn
3a' flache Flanke des Zahns
3b Aussengewinde
3c Gewindestangeanschlag
- 4: Dosierhülse
4a Dosierhülsensteg
4b Dosierhülsengewinde
4c Dosierhülsenringnut
- 5: Dosierknopf
- 6: Gehäuse
6a Gehäusehaltearm
6b Gehäusefenster
6c Nut
6d Gehäuseführungssteg
6e Gehäusesteg
- 7: Flansch
- 8: Karpulenhalter
8a Nadelverbindungselement
8b Karpulenhaltervorsprung
8c Ausnehmung
8d Karpulenhaltergehäuse
8e Karpulenhalterführungsnut
- 9, 9', 9": Halteelement
9a Vorsprung
9b, 9b', 9b" Nocke
9c Federelement
9d, 9d', 9d" Klemmelement
9e, 9e', 9e" Ringelement
9f, 9f' erster Federsitz
9g, 9g' zweiter Federsitz
9h, 9h' Bügel
9i, 9i' Verbindungsstege
- 10: Karpule
10a proximaler Karpulenrand
10b Stopfen
- 11: Verriegelungsring
11a Ratschenzahn
11b Riegelarm
11c Kupplungsverzahnung
11d Anschlagfläche
- 12: Klebstoff

## Patentansprüche

1. Karpulenhalter (8) zur Aufnahme einer Karpule (10) und zur Befestigung an eine Antriebs- und/oder an eine Dosiereinrichtung und/oder an einem Gehäuse (6) einer Injektionsvorrichtung umfassend:
- ein Halteelement (9, 9', 9") zur Befestigung der Karpule (10) an oder in dem Karpulenhalter (8),
- wobei das Halteelement (9, 9', 9") derart ausgebildet ist, dass es mit dem Karpulenhalter (8) verbindbar ist und von dem Karpulenhalter (8) vollständig aufnehmbar ist, wobei das Halteelement (9, 9', 9") elastisch verformbar ausgebildet ist und
- das Halteelement (9, 9', 9") eine Nocke (9b, 9b', 9b") umfasst, um das Haltelement (9, 9', 9") elastisch vorzuspannen, wenn die Nocke (9b, 9b', 9b") des Halteelements in Eingriff mit einer Ausnehmung (8c) des Karpulenhalters (8) ist und
- das Halteelement (9, 9', 9") ein Klemmelement (9d, 9d', 9d") umfasst, wobei sich das Klemmelement (9d, 9d', 9d") radial nach innen erstrecken kann, um die in den Karpulenhalter (8) eingesetzte Karpule (10) zu halten oder zu umgreifen oder einzuklemmen.

2. Karpulenhalter (8) nach Anspruch 1, **dadurch gekennzeichnet, dass** das Haltelement (9) einen Vorsprung (9a) und ein Federelement (9c) umfasst.

3. Karpulenhalter (8) nach Anspruch 2, **dadurch gekennzeichnet, dass** an dem Federelement (9c) der Vorsprung (9a) und das Klemmelement (9d) vorgesehen sind.

4. Karpulenhalter (8) nach einem der vorhergehenden Ansprüche, **dadurch gekennzeichnet, dass** der Karpulenhalter (8) ein Karpulenhaltergehäuse (8d) umfasst.

5. Karpulenhalter (8) nach Anspruch 4, **dadurch gekennzeichnet, dass** das Karpulenhaltergehäuse (8d) des Karpulenhalters (8) eine Ausnehmung (8c) aufweist.

6. Karpulenhalter (8) nach einem der vorhergehenden Ansprüche, **dadurch gekennzeichnet, dass** das Haltelement (9, 9', 9") ein Ringelement (9e, 9e', 9e") umfasst.

7. Karpulenhalter (8) nach Anspruch 6, **dadurch gekennzeichnet, dass** das Ringelement (9e, 9e', 9e") einen ersten Federsitz (9f, 9f) und einen zweiten Federsitz (9g, 9g') umfasst.

8. Karpulenhalter (8) nach Anspruch 7, **dadurch gekennzeichnet, dass** an einem der Federsitze (9g, 9g') die Nocke (9b, 9b') vorgesehen ist.

9. Karpulenhalter (8) nach Anspruch 7 oder 8, **dadurch gekennzeichnet, dass** das Ringelement (9e, 9e') mehrere Bügel (9h, 9h') und mehrere Verbindungsstege (9i, 9i') umfasst, wobei der erste Federsitz (9f, 9f) über die mehreren Bügel (9h, 9h') und über die mehreren Verbindungsstege (9i, 9i') mit dem zweiten Federsitz (9g, 9g') verbunden ist.

10. Karpulenhalter (8) nach einem der vorhergehenden Ansprüche, **dadurch gekennzeichnet, dass** in einer ersten Position, bei welcher die Karpule (10) im Karpulenhalter (8) bewegbar aufgenommen ist, das Halteelement (9, 9', 9") zumindest teilweise in einem Ringspalt zwischen dem Karpulenhalter (8) und der Karpule (10) angeordnet ist.

11. Karpulenhalter (8) nach den Ansprüchen 5 und 10, **dadurch gekennzeichnet, dass** in der ersten Position der Vorsprung (9a) des Halteelements (9, 9', 9") in die Ausnehmung (8c) des Karpulenhalters (8) ragt, um das Halteelement (9, 9', 9") mit dem Karpulenhalter (8) zu verbinden.

12. Karpulenhalter (8) nach einem der vorhergehenden Ansprüche, **dadurch gekennzeichnet, dass** in einer zweiten Position, bei welcher die Karpule (10) im Karpulenhalter (8) aufgenommen und befestigt ist, das Halteelement (9, 9', 9") zumindest teilweise in einem Ringspalt zwischen dem Karpulenhalter (8) und der Karpule (10) angeordnet ist.

13. Karpulenhalter (8) nach Anspruch 12, **dadurch gekennzeichnet, dass** das Halteelement (9, 9', 9") derart ausgebildet ist, dass in der zweiten Position das Halteelement (9, 9', 9") die Karpule (10) relativ zu dem Karpulenhalter (8) axial spielfrei halten kann.

14. Karpulenhalter (8) nach den Ansprüchen 5, 9 und 13, **dadurch gekennzeichnet, dass** in der zweiten Position die Nocke (9b, 9b', 9") des Halteelements (9, 9') in die Ausnehmung (8c) des Karpulenhalters (8) ragt, um das Halteelement (9, 9') elastisch vorzuspannen.

15. Karpulenhalter (8) nach den Anspruch 14, **dadurch gekennzeichnet, dass** das Klemmelement (9d, 9d', 9d") des Halteelements (9, 9', 9") in der zweiten Position einen proximalen Karpulenrand (10a) hält oder umgreift.

16. Verfahren zur Montage einer Karpule (10) an oder in einen Karpulenhalter (8) nach einem der vorhergehenden Ansprüche umfassend folgende Schritte:
- Einbringen eines Vorsprungs (9a) des Halteelements (9) in eine Ausnehmung (8c) des Karpulenhalters (8)
- Einsetzen der Karpule (10) in den Karpulenhalter (8)
- Verschieben des Halteelements (9) in distaler Richtung, sodass der Vorsprung (9a) des Halteelements (9) aus der Ausnehmung (8c) des Karpulenhalters (8) und eine Nocke (9b) des Halteelements (9) in die Ausnehmung (8c) des Karpulenhalters (8) gelangt, um das Halteelement (9) elastisch vorzuspannen derart, dass die Karpule (10) spielfrei mit dem Karpulenhalter (8) verbunden ist, und wobei ein Klemmelement (9d) des Halteelements (9) ein proximalen Karpulenrands (10a) hält oder umgreift.

## Claims

1. A carpule holder (8) for receiving a carpule (10) and for fixing to a drive device and/or to a dosing device and/or to a housing (6) of an injection device, including:
a holding element (9, 9', 9") for fixing the carpule (10) to or in the carpule holder (8),
wherein the holding element (9, 9', 9") is of such a configuration that it can be connected to the carpule holder (8) and can be completely received by the carpule holder (8), the holding element (9, 9', 9") being elastically deformable, and
the holding element (9, 9', 9") includes a cam (9b, 9b', 9b") to elastically prestress the holding element (9, 9', 9") when the cam (9b, 9b', 9b") of the holding element is in engagement with a recess (8c) in the carpule holder (8), and
the holding element (9, 9', 9") includes a clamping element (9d, 9d', 9d"), wherein the clamping element (9d, 9d', 9d") can extend radially inwardly in order to hold or embrace or clamp the carpule (10) fitted into the carpule holder (8).

2. A carpule holder (8) according to claim 1 **characterised in that** the holding element (9) includes a projection (9a) and a spring element (9c).

3. A carpule holder (8) according to claim 2 **characterised in that** the projection (9a) and the clamping element (9d) are provided on the spring element (9c).

4. A carpule holder (8) according to one of the preceding claims **characterised in that** the carpule holder (8) includes a carpule holder housing (8d).

5. A carpule holder (8) according to claim 4 **characterised in that** the carpule holder housing (8d) of the carpule holder (8) has a recess (8c).

6. A carpule holder (8) according to one of the preceding claims **characterised in that** the holding element (9, 9', 9") includes a ring element (9e, 9e', 9e").

7. A carpule holder (8) according to claim 6 **characterised in that** the ring element (9e, 9e', 9e") includes a first spring seat (9f, 9f') and a second spring seat (9g, 9g').

8. A carpule holder (8) according to claim 7 **characterised in that** the cam (9b, 9b') is provided on one of the spring seats (9g, 9g').

9. A carpule holder (8) according to claim 7 or claim 8 **characterised in that** the ring element (9e, 9e') has a plurality of hoops (9h, 9h') and a plurality of connecting bars (9i, 9i'), wherein the first spring seat (9f, 9f') is connected to the second spring seat (9g, 9g') by way of the plurality of hoops (9h, 9h') and by way of the plurality of connecting bars (9i, 9i').

10. A carpule holder (8) according to one of the preceding claims **characterised in that** in a first position in which the carpule (10) is moveably received in the carpule holder (8) the holding element (9, 9', 9") is at least partially arranged in an annular gap between the carpule holder (8) and the carpule (10).

11. A carpule holder (8) according to claims 5 and 10 **characterised in that** in the first position the projection (9a) of the holding element (9, 9', 9") projects into the recess (8c) of the carpule holder (8) to connect the holding element (9, 9' 9") to the carpule holder (8).

12. A carpule holder (8) according to one of the preceding claims **characterised in that** in a second position in which the carpule (10) is received and fixed in the carpule holder (8) the holding element (9, 9', 9") is at least partially arranged in an annular gap between the carpule holder (8) and the carpule (10).

13. A carpule holder (8) according to claim 12 **characterised in that** the holding element (9, 9', 9") is of such a configuration that in the second position the holding element (9, 9', 9") can hold the carpule (10) in axially play-free relationship relative to the carpule holder (8).

14. A carpule holder (8) according to claims 5, 9 and 13 **characterised in that** in the second position the cam (9b, 9b', 9b") of the holding element (9, 9") projects into the recess (8c) in the carpule holder (8) to elastically prestress the holding element (9, 9').

15. A carpule holder (8) according to claim 14 **characterised in that** the clamping element (9d, 9d', 9d") of the holding element (9, 9', 9") holds or embraces a proximal carpule edge (10a) in the second position.

16. A method of fitting a carpule (10) to or in a carpule holder (8) according to one of the preceding claims including the following steps:
- introducing a projection (9a) of the holding element (9) into a recess (8c) of the carpule holder (8),
- inserting the carpule (10) into the carpule holder (8),
- displacing the holding element (9) in the distal direction so that the projection (9a) of the holding element (9) passes out of the recess (8c) of the carpule holder (8) and a cam (9b) of the holding element (9) passes into the recess (8c) in the carpule holder (8) to elastically prestress the holding element (9) in such a way that the carpule (10) is play-free connected to the carpule holder (8) and wherein a clamping element (9d) of the holding element (9) holds or embraces a proximal carpule edge (10a).

## Revendications

1. Support de carpule (8) destiné à recevoir une carpule (10) et destiné à être fixé à un système d'entraînement et/ou à un système de dosage et/ou sur un boîtier (6) d'un dispositif d'injection, comprenant :
- un élément de maintien (9, 9', 9") pour fixer la carpule (10) sur ou dans le support de carpule (8),
- dans lequel l'élément de maintien (9, 9', 9") est réalisé pour qu'il puisse être relié au support de carpule (8) et puisse être reçu en totalité par le support de carpule (8), dans lequel l'élément de maintien (9, 9', 9") est réalisé de manière déformable élastiquement, et
- l'élément de maintien (9, 9', 9") comprend une came (9b, 9b', 9b") pour précontraindre élastiquement l'élément de maintien (9, 9', 9") quand la came (9b, 9b', 9b") de l'élément de maintien est en prise avec un évidement (8c) du support de carpule (8), et
- l'élément de maintien (9, 9', 9") comprend un élément de serrage (9d, 9d', 9d"), dans lequel l'élément de serrage (9d, 9d', 9d") peut s'étendre radialement vers l'intérieur pour maintenir ou entourer ou coincer la carpule (10) insérée dans le support de carpule (8).

2. Support de carpule (8) selon la revendication 1, **caractérisé en ce que** l'élément de maintien (9) comprend une partie faisant saillie (9a) et un élément de ressort (9c).

3. Support de carpule (8) selon la revendication 2, **caractérisé en ce que** la partie faisant saillie (9a) et l'élément de serrage (9d) sont prévus sur l'élément de ressort (9c).

4. Support de carpule (8) selon l'une quelconque des revendications précédentes, **caractérisé en ce que** le support de carpule (8) comprend un boîtier de support de carpule (8d).

5. Support de carpule (8) selon la revendication 4, **caractérisé en ce que** le boîtier de support de carpule (8d) du support de carpule (8) présente un évidement (8c).

6. Support de carpule (8) selon l'une quelconque des revendications précédentes, **caractérisé en ce que** l'élément de maintien (9, 9', 9") comprend un élément annulaire (9e, 9e', 9e").

7. Support de carpule (8) selon la revendication 6, **caractérisé en ce que** l'élément annulaire (9e, 9e', 9e") comprend un premier siège de ressort (9f, 9f') et un deuxième siège de ressort (9g, 9g').

8. Support de carpule (8) selon la revendication 7, **caractérisé en ce que** la came (9b, 9b') est prévue sur un des sièges de ressort (9g, 9g').

9. Support de carpule (8) selon la revendication 7 ou 8, **caractérisé en ce que** l'élément annulaire (9e, 9e') comprend plusieurs arceaux (9h, 9h') et plusieurs entretoises de liaison (9i, 9i'), dans lequel le premier siège de ressort (9f, 9f') est relié au deuxième siège de ressort (9g, 9g') par l'intermédiaire des plusieurs arceaux (9h, 9h') et par l'intermédiaire des plusieurs entretoises de liaison (9i, 9i').

10. Support de carpule (8) selon l'une quelconque des revendications précédentes, **caractérisé en ce que**, dans une première position, dans laquelle la carpule (10) est reçue de manière à pouvoir être déplacée dans le support de carpule (8), l'élément de maintien (9, 9', 9") est disposé au moins en partie dans une fente annulaire entre le support de carpule (8) et la carpule (10).

11. Support de carpule (8) selon les revendications 5 et 10, **caractérisé en ce que**, dans la première position, la partie faisant saillie (9a) de l'élément de maintien (9, 9', 9") dépasse dans l'évidement (8c) du support de carpule (8) pour relier l'élément de maintien (9, 9', 9") au support de carpule (8).

12. Support de carpule (8) selon l'une quelconque des revendications précédentes, **caractérisé en ce que**, dans une deuxième position, dans laquelle la carpule (10) est reçue et fixée dans le support de carpule (8), l'élément de maintien (9, 9', 9") est disposé au moins en partie dans une fente annulaire entre le support de carpule (8) et la carpule (10).

13. Support de carpule (8) selon la revendication 12, **caractérisé en ce que** l'élément de maintien (9, 9', 9") est réalisé de telle manière que, dans la deuxième position, l'élément de maintien (9, 9', 9") peut maintenir axialement sans jeu la carpule (10) par rapport au support de carpule (8).

14. Support de carpule (8) selon les revendications 5, 9 et 13, **caractérisé en ce que**, dans la deuxième position, la came (9b, 9b', 9") de l'élément de maintien (9, 9') dépasse dans l'évidement (8c) du support de carpule (8) pour précontraindre de manière élastique l'élément de maintien (9, 9').

15. Support de carpule (8) selon la revendication 14, **caractérisé en ce que** l'élément de serrage (9d, 9d', 9d") de l'élément de maintien (9, 9', 9") maintient ou entoure un bord de carpule proximal (10a) dans la deuxième position.

16. Procédé de montage d'une carpule (10) sur ou dans un support de carpule (8) selon l'une quelconque des revendications précédentes, comprenant les étapes suivantes :
- d'introduction d'une partie faisant saillie (9a) de l'élément de maintien (9) dans un évidement (8c) du support de carpule (8),
- d'insertion fde la carpule (10) dans le support de carpule (8),
- de coulissement de l'élément de maintien (9) dans une direction distale de telle sorte que la partie faisant saillie (9a) de l'élément de maintien (9) sort de l'évidement (8c) du support de carpule (8) et une came (9b) de l'élément de maintien (9) parvient dans l'évidement (8c) du support de carpule (8) pour précontraindre de manière élastique l'élément de maintien (9) de telle manière que la carpule (10) est reliée sans jeu au support de carpule (8), et dans lequel un élément de serrage (9d) de l'élément de maintien (9) maintient ou entoure un bord de carpule proximal (10a).
